# EUROPÄISCHE PATENTSCHRIFT

(11) **EP 2 334 768 B1**
(45) Veröffentlichungstag und Bekanntmachung des Hinweises auf die Patenterteilung: **16.03.2016**
(21) Anmeldenummer: 09776150.6
(22) Anmeldetag: 24.08.2009
(51) Int. Cl.: C10L 3/06, C10L 3/08

(54) **VERFAHREN ZUR BIOGASAUFBEREITUNG UND BIOGASANLAGE**
METHOD FOR BIOGAS TREATMENT AND BIOGAS INSTALLATION
PROCÉDÉ DE TRAITEMENT DE BIOGAZ ET INSTALLATION DE PRODUCTION DE BIOGAZ

(30) Priorität: 11.09.2008 DE 102008046879
(43) Veröffentlichungstag der Anmeldung: 22.06.2011
(73) Patentinhaber: Schmack Biogas GmbH, 92421 Schwandorf (DE)
(72) Erfinder: WOLF, Markus, 92431 Neunburg vorm Wald (DE); NETTELNBREKER, Ulrich, 48619 Heek (DE)
(74) Vertreter: Glück Kritzenberger Patentanwälte PartGmbB
(86) Internationale Anmeldenummer: PCT/DE2009/075044
(87) Internationale Veröffentlichungsnummer: WO 2010/028643

(56) Entgegenhaltungen:
- EP-A1- 1 811 011
- AT-B- 410 273
- DE-A1- 10 047 264
- DE-A1- 19 947 339

## Beschreibung

Die Erfindung betrifft ein Verfahren zur Biogasaufbereitung, bei dem das aus einer Fermentation stammende Gas in einen nutzbaren, im wesentlichen aus Methangas bestehenden Biogasstrom und einen Störstoffe (im wesentlichen Kohlendioxid) enthaltenden Abgasstrom getrennt wird, der thermisch oder katalytisch oxidiert wird.

Die Erfindung betrifft ferner eine Biogasanlage mit einem oder mehreren Fermentationsbehältern und gegebenenfalls Nachgärern, einer Biogasaufbereitungsanlage zur Trennung von Biogas und Abgas, wobei eine Abgasführung eine abschließende thermische oder katalytische Oxidationsstufe aufweist, sowie mindestens mit einem Substratlagerbehälter und/oder Gärrestbehälter.

Durch anaerobe Vergärung von organischen Stoffen entsteht u. a. Methan, welches ein hochwertiger Energieträger ist, der unter Ausnutzung seines Energiegehaltes beispielsweise zu Heizzwecken verwertbar ist. Weitere Gasbestandteile, die im Rahmen einer Fermentation entstehen, sind Kohlendioxid, Schwefelwasserstoff, Stickstoff und andere Störstoffe. Es ist bekannt bei einer Biogasaufbereitung einen im wesentlichen Methan enthaltenden Gasstrom abzutrennen, der unmittelbar ins Gasnetz eingespeist werden kann. Für die Aufbereitung von Rohbiogasen aus einer Fermentation müssen eine Biogasentschwefelung, eine Gastrocknung und insbesondere eine Kohlendioxidabtrennung herbeigeführt werden. Eine Kohlendioxidabtrennung kann nach dem Stand der Technik beispielsweise über eine Gaswäsche mittels Waschflüssigkeit (z.B. Wasser, Polyglykol, MEA-Wäsche), über eine Druckwechseladsorption (DWA), über ein Membrantrennverfahren (nass oder trocken), oder eine Kohlendioxidverflüssigung durchgeführt werden.

Aus ökologischen und auch aus wirtschaftlichen Gründen müssen bei der Rohbiogasaufbereitung Methanverluste gering gehalten werden. Ebenso sind Methan- oder Kohlendioxidemissionen in die Atmosphäre wegen Klimaschutzabkommen zum Treibhauseffekt zu vermeiden. Die nach dem Stand der Technik bekannten Kohlendioxid-Abtrennungsverfahren weisen technisch bedingt einen Methanverlust auf, der abhängig von dem angewendeten CO₂-Abtrennungsverfahren zwischen 2 und 6 Vol% (da es sich bei allen in der Biogasanlage vorkommenden Gasen um weitgehend "ideale Gase" handelt, können die Angaben Vol% jeweils mit Mol% gleichgesetzt werden) liegt. Deshalb muss der Abgasstrom bei fast allen Biogasaufbereitungsverfahren einer Abgasbehandlung unterzogen werden.. In der Fachwelt wird grundsätzlich unterschieden zwischen dem Methanverlust, also der Methanmenge, die nicht ins Gasnetz eingespeist werden kann, und der Methankonzentration im Abgasstrom. Beispielsweise hat bei einer Aufbereitung von Biogas mit einem Methangehalt von 53 Vol% und einem Methanverlust von 3 Vol% sowie einem Methangehalt im einzuspeisenden Produktgas von 96 Vol% das Abgas eine Methankonzentration von 3,42 Vol%. Um die Freisetzung von Methan in die Umwelt aus Biogasanlagen zu minimieren, hat der Gesetzgeber finanzielle Fördermaßnahmen eingerichtet (z.B. "Technologie-Bonus" aufgrund des EEG (Erneuerbare-Energien-Gesetz) in Deutschland), bei denen jedoch die maximal emittierbare Methanmenge im Abgas einer Biogasaufbereitung auf 0,5 Vol% der im Biogasprozess produzierten Methanmasse begrenzt wird. Um die in dem oben genannten Beispiel aufgeführte Methankonzentration auf den geforderten Wert von 0,5 Vol% zu reduzieren, wird üblicherweise das Abgas thermisch oder katalytisch oxidiert. Hierzu sind sog. FLOX-Brenner, d. h. Brenner mit einer flammenlosen Oxidation oder die katalytische Nachverbrennung (KNV) bekannt. Um die Nachverbrennung durchführen zu können, muss gegebenenfalls Biogas, Erdgas oder Flüssiggas beigemengt werden.

Aus der EP 1 811 011 A1 ist ein Verfahren zur Gewinnung von Methan aus Deponiegas bekannt. Durch Entfernung der in dem Biogas störenden Komponenten wie Schwefelwasserstoff, Sauerstoff, Stickstoff, Kohlendioxid, Wasser und flüchtige Fettsäuren soll möglichst reines Methan erhalten werden, welches dann weiter genutzt werden kann.

Die DE 100 47 264 A1 stellt ein Verfahren zur Nutzung von methanhaltigem Biogas, insbesondere von Deponiegas und Biogas aus Vergärungsanlagen oder Faulprozessen auf Kläranlagen zur Verfügung. Das Biogas wird in zwei Gasströme getrennt, von denen der eine einen höheren Methangehalt als Biogas aufweist und der andere mit Kohlendioxid angereichert ist. Der mit Kohlendioxid angereicherte Gasstrom wird in den Biogas erzeugenden Prozess zurückgeführt.

Die DE 199 47 339 A1 offenbart ein Verfahren, bei dem eine Mischung aus Energiepflanzen und wenigstens einer der Biomassen Gülle und organischer Abfall mittels Mikroorganismen fermentativ anaerob abgebaut wird und aus einem bei dem Abbau entstehenden Biogasgemisch erdgasgleiches Biomethan und Kohlendioxid hergestellt werden. Aus dem Rohbiogas werden mit Hilfe von Gastrennverfahren methanreiche bzw. kohlendioxidreiche Teilströme erzeugt. Der CO₂-reiche Gasstrom kann als Siliergas eingesetzt werden, wobei das CO₂ durch am Siloboden liegende Leitungen in die zu silierende Schüttung eingepresst wird. Außerdem wird die Durchleitung des CO₂-reichen Gases durch Schutzräume über einem Nachgärtank und einem Tank für entschwefeltes Biogas offenbart. Dadurch wird ein Luft- oder Sauerstoffzutritt verhindert, wodurch die Reinheit und damit Vermarktungsfähigkeit der Gase gewährleistet bleibt.

Darüber hinaus ist es nach dem Stand der Technik ebenfalls bekannt, die für die Vergärung eingesetzten organischen Stoffe vor der Vergärung aufzubereiten oder zwischen zu lagern. So ist es durchaus üblich, feste Stoffe entweder direkt in die Vergärungsstufe einzutragen oder über unterschiedliche Systeme einzuspülen, wohingegen flüssige Rohstoffe wie Gülle oder andere pumpfähige organische Abfälle zumeist in einem oder mehreren Substratlagerbehältern gepuffert werden. In solchen Substratlagerbehältern können auch Rezirkulate, d. h. Substrate aus unterschiedlichen Anlagenbereichen und/oder aus einer Separation rückgeführt werden, bevor sie einem oder mehreren Fermentern und anschließend vorhandenen Nachgärbehältern zugeführt werden. Vorhandener Gärrest wird in sog. Gärrestbehältern oder Substratlagerbehältern oder Endlagern zur weiteren Behandlung oder zur Ausbringung auf landwirtschaftliche Nutzflächen zwischengelagert. In den Substratlagerbehältern und den Gärrestbehältern entstehen Gase, deren Art und Zusammensetzung von der biologischen Aktivität und dem organischen Abbaugrad der in den Behältern gelagerten Substanzen abhängt. Während die Biogasentstehung im Fermenter oder in Nachgärbehältern erwünscht ist und durch die eingestellten Bedingungen gefördert wird, wirkt sich eine Gasbildung in Substratlagerbehältern und Gärrestbehältern störend aus. Auch durch das Anmischen von frischen relativ trockenen organischen Stoffen wie z. B. Silagen mit aktivem Fermenterinhalt oder durch das Einleiten der flüssigen Phase aus der Fest/Flüssigseparation von aktiven Fermentermaterial in den Fermentationsprozess vor- und nachgelagerten Behältern wird eine störende Gasbildung erzeugt.

Nach dem bisherigen Stand der Technik sind Substratlagerbehälter und/oder Gärrestbehälter z. T. offen, z. T. ohne eine gasdichte Abdeckung ausgeführt worden, so dass die entstehenden Gase ungehindert in die Atmosphäre geströmt sind. Die emittierten Gasgemische enthalten nicht nur geruchsintensive Gase wie z. B. Schwefelwasserstoff, Ammoniak und Mercaptane bzw. Alkanthiole, sondern insbesondere auch Kohlendioxid und Methan, die zwar geruchlos sind, jedoch als Hauptverursacher der anthropogenen Klimaerwärmung gelten.

Methan stellt ein Treibhausgas dar, das etwa 25mal wirksamer ist als Kohlendioxid und somit global als eines der für den Treibhauseffekt verantwortlichen Gase gilt. Bereits eine Oxidation des Methans zu Kohlendioxid und Wasser führt zu einer Herabsenkung des Treibhauseffektes um den Faktor 25. Hierin liegt auch der Grund, weshalb neue gesetzliche Bestimmungen fordern, den Methangasanteil in Emissionen aus Biogasanlagen zu reduzieren. Z. T. werden gesetzliche Anreize zu einer Methanemissionsreduzierung dadurch geschaffen, dass ansonsten gewährbare Fördermittel entfallen, sofern die Biogasanlage nicht gasdicht abgedichtete Behälter besitzt. Die gasdichte Abdeckung von Gärrestbehältern und Substratlagerbehälter führt jedoch zu neuen Problemen, da die entstehenden Methangase bei Normaldruck in Kontakt mit Luft bei Umgebungstemperatur ab einer gewissen Konzentration explosiv sind, so dass ein zusätzlicher Aufwand für einen Explosionsschutz notwendig ist. Die größte Gefahr besteht dann, wenn zu Zeiten einer Gärrestabfuhr die Gärrestbehälter entleert werden, so dass der durch diese Entleerung entstehende Raum große Kapazitäten explosiver Gase aufnehmen kann. Die gesetzlichen Anforderungen an Biogasanlagen zur Vermeidung von Explosionen führen jedoch dazu, dass Explosionsschutzmaßnahmen die Biogaserzeugung insgesamt teurer machen. In Deutschland kommen für eine Biogasanlage beispielsweise folgende Verordnungen zum Tragen:
- "Vierte Verordnung zur Durchführung des Bundes-Immissionsschutzgesetzes (Verordnung über genehmigungsbedürftige Anlagen - 4.BImSchV)";
- "Zwölfte Verordnung zur Durchführung des Bundes-Immissionsschutzgesetzes (Störfall-Verordnung - 12.BImSchV)" und
- "Sicherheitsregeln für landwirtschaftliche Biogasanlagen", Schutzabstände nach Punkt 2.4.5.4 Ballon- und Kissenspeicher sowie Folienspeicher über Güllelager oder Gärbehälter.

In anderen Ländern existieren vergleichbare Verordnungen.

Es ist Aufgabe der vorliegenden Erfindung, ein Verfahren zur Biogasaufbereitung sowie eine Biogasanlage zu schaffen, die einen hinreichenden Schutz vor Explosionen liefern und dennoch einfach aufgebaut ist, so dass ein optimaler wirtschaftlicher Betrieb möglich ist.

Diese Aufgabe wird durch das Verfahren nach Anspruch 1 sowie die Biogasanlage nach Anspruch 4 gelöst. Weiterentwicklungen sind in den Unteransprüchen beschrieben.

Der Kerngedanke der vorliegenden Erfindung besteht darin, dass der Abgasstrom, der aus der Biogasaufbereitung stammt, vor der abschließenden Oxidation durch die geschlossen ausgebildeten Substratlagerbehälter und/oder Gärrestbehälter geleitet wird. Dieser Abgasstrom, der im wesentlichen aus Kohlendioxid besteht und nur geringe Methangehalte aufweist, dient somit zur Inertisierung der Gasvolumina der Substratlagerbehälter und/oder Gärrestbehälter. Diese freien Volumina werden somit von dem Abgasstrom durchflutet, der die Entstehung von explosiven Gasgemischen bzw. das Überschreiten von Explosionen fördernden Konzentrationen verhindert. Dieser Spüleffekt kann ohne großen Aufwand betrieben werden und verhindert Investitionen für sonst gesetzlich vorgeschriebene Explosionsschutzmaßnahmen.

Der Abgasstrom kann vorhandene Substratlagerbehälter und/oder Gärrestbehälter, die jeweils ansonsten gasdicht verschlossen sind, vorzugsweise nacheinander durchströmen. Diese Ausgestaltung hat den Vorteil, dass nur am letzten durchströmten Behälter ein Gasspeichervolumen (Arbeitsvolumen) geschaffen werden muss, der zur Steuerung der thermischen oder katalytischen Oxidation erforderlich ist und füllstandsüberwacht werden kann.

Ein weiterer Vorteil besteht darin, dass die Gasräume aller Behälter kontinuierlich mit dem gesamten Abgasstrom durchspült werden und sich somit keine Bereiche bilden können, in denen es zu Aufkonzentrierungen von Methan kommen kann.

Die vorliegende Erfindung umfasst also ein Verfahren zur Biogasaufbereitung, bei dem das aus einer Fermentation stammende Biogas in einen direkt nutzbaren, im wesentlichen aus Methangas bestehenden Gasstrom und einen Störstoffe enthaltenden Abgasstrom getrennt wird, der thermisch oder katalytisch oxidiert wird. Der Abgasstrom wird vor der Oxidation durch geschlossen ausgebildete Substratlagerbehälter und/oder Gärrestbehälter durchgeleitet.

Unter einen "im wesentlichen aus Methangas bestehenden Gasstrom" wird im Rahmen der vorliegenden Erfindung ein Gasstrom verstanden, der zumindest 90 Vol% Methan, bevorzugt zumindest 95 Vol% Methan enthält.

Selbstverständlich ist auch eine parallele Durchströmung der Behälter möglich, was es erforderlich macht, dass der Abgasstrom aus der Biogasaufbereitung in entsprechend viele Teilströme wie Behälter durchströmt werden müssen aufgeteilt wird, so dass jeder Substratlagerbehälter und/oder Gärrestbehälter eine entsprechende Gaszufuhr in ausreichendem Maße erhält. Die Gasauslässe aller vorhandenen Behälter führen in eine gemeinsame Abgasleitung, die zur thermischen oder katalytischen Oxidationsstufe führt.

Vorzugsweise wird das Abgas abschließend verbrannt und die Verbrennungswärme zur Deckung des Wärmebedarfs der Biogasanlage und ggf. externer Wärmeverbraucher genutzt.

Im Gegensatz zu Verfahren, die aus dem Stand der Technik bekannt sind, wird der hauptsächlich CO₂ sowie geringe Mengen Methan enthaltende Abgasstrom nicht durch Fermentationsbehälter, Nachgärer oder Bioreaktoren geleitet, die der Erzeugung von Biogas dienen, sondern lediglich durch gasdicht abgedeckte Behälter einer Biogasanlage wie Substratlagerbehälter und/oder Gärrestbehälter, bei denen eine Produktion von Methan und CO₂-haltigem Gas im Grunde unerwünscht ist, da sich dadurch Probleme mit klimaschädlichen Umweltemissionen und sicherheitstechnischer Art ergeben. Der hauptsächlich CO₂ sowie geringe Mengen Methan enthaltende Abgasstrom wird nicht in flüssige Phasen mit einem Feststoffanteil, die Substrat enthalten, eingeleitet, sondern lediglich durch andere gasförmige Phasen geleitet, um in erfindungsgemäßer Weise die Konzentrationen der dort vorhandenen Gasmischungen zu verändern. Die erfindungsgemäße Behandlung des Abgasstromes dient zwar nicht zur Erhöhung der Methanausbeute im bei der Fermentation erzeugten Biogas, ein positiver Effekt auf die Energiebilanz der gesamten Anlage entsteht jedoch dadurch, dass das im Abgasstrom enthaltene Methan sowie das in Substratlagerbehältern und Gärrestbehältern entstandene Methan durch nachgeschaltete Oxidation in thermische Energie umgewandelt werden kann, die ebenfalls genutzt werden kann (z.B. zur Deckung des Wärembedarfs der Biogasanlage selbst).

Die erfindungsgemäß in den Substratlagerbehältern sowie den Gärrestbehältern bewirkte Minimierung der Masse brennbarer und hoch entzündlicher Gase reduziert aufwendige Explosionsschutzmaßnahmen.

Der Abgasstrom von Biogasaufbereitungsanlagen weist in der Regel einen Methangehalt von 2,3 bis 6,4 Vol% und einen Kohlendioxidgehalt von 96,7 Vol% bis 93,6 Vol% auf. Der Rest sind geringe Anteile von Stickstoff, Sauerstoff, Wasserstoff und Schwefelwasserstoff. Methangasgemische können unter Normaldruck bei 20 ° C mit einem Methangasgehalt von 4,3 Vol% als unterer Explosionsgrenze und 16,3 Vol% als oberer Explosionsgrenze explosiv sein (bei 100 °C untere Grenze 4,0 %, obere Grenze 17,3 %), allerdings nur, wenn der Inertgasanteil (z.B. CO₂) unter 30,5 Vol% liegt (bei 100 °C Inertgasanteil mindestens 33,5 Vol%). Weitere Ausführungen zu den Explosionsbereichen von Methangasgemischen sind dem Fachmann aus Diagrammen mit einer Darstellung in Dreieckskoordinaten bekannt. Das Abgas der Biogasaufbereitungsanlage mit seinem hohen Inertgasanteil von größer 90 Vol% ist somit nicht explosiv und kann zur Inertisierung der Freiräume im Substratlagerbehälter und in den Gärrestbehälter genutzt werden. Eine Verdrängung der vorhandenen Luft durch den Abgasstrom zur Inertisierung der Freiräume im Substratlagerbehälter und in den Gärrestbehälter wird bereits vor Befüllung der entsprechenden Behälter mit Gärsubstrat bzw. Gärresten durchgeführt. Im Betrieb der Anlage dient die kontinuierliche Inertisierung der Gasbereiche in Substratlagerbehälter und in den Gärrestbehältern dazu, dort in geringem Maße entstehendes Biogas mit hohem Methangehalt zu verdünnen und jeweils einen hohen Gehalt an Inertgas (hier CO₂) aufrecht zu erhalten, so dass der Explosionsschutz gewährleistet werden kann. Der Grenzwert von 17,5 Vol% Methan, bei dem ein Methangasgemisch bei Normaldruck, nachdem es mit Luft in Kontakt gekommen und dadurch verdünnt worden ist, bei Umgebungstemperatur entzündlich ist, bleibt stets deutlich unterschritten.

Die bis auf den Zu- und -Ablauf des Abgasstroms geschlossene Behälterausbildung vermeidet zudem Geruchsemissionen sowie das Ausströmen von Methan und/oder Kohlendioxid. Der Abgasstrom, der parallel oder in Serie die Substratlagerbehälter und Gärrestbehälter durchströmt hat, wird abschließend oxidiert, insbesondere verbrannt. Hierbei dienen das im Behälter gebildete Methan sowie der Methanschlupf der Gasaufbereitung als Brennstoff. Die überschüssige thermische Leistung der exothermen Reaktion in der Abgasbehandlung wird in einem Heizsystem ausgekoppelt und kann z. B. zur Deckung des Wärmebedarfs der Biogasanlage und ggf. anderer Wärmeverbraucher benutzt werden. Nach dem erfindungsgemäßen Verfahren wird hierbei nicht nur der Energiegehalt des Methananteils aus dem Abgasstrom, also im Prinzip der Methanverlust bei der Biogasaufbereitung, sondern auch der Energiegehalt des Methananteils aus der zusätzlichen - vergleichsweise geringen - Biogasproduktion in den Substratlagerbehältern und den Gärrestbehältern genutzt. Die Nutzung dieser Methananteile ist ökonomisch sinnvoll, da sich die erfindungsgemäße Nutzung des Abgasstromes technisch ohne großen Aufwand realisieren lässt.

Ausführungsbeispiele der vorliegenden Erfindung sind in den Zeichnungen sowie weiteren Beispielen zur technischen Ausführung dargestellt. Es zeigen
- Fig. 1 und 2: jeweils Prinzipskizzen der Biogas- und Abgasführung und
- Fig. 3: eine Skizze einer erfindungsgemäßen 6,5 MW Biogasanlage.

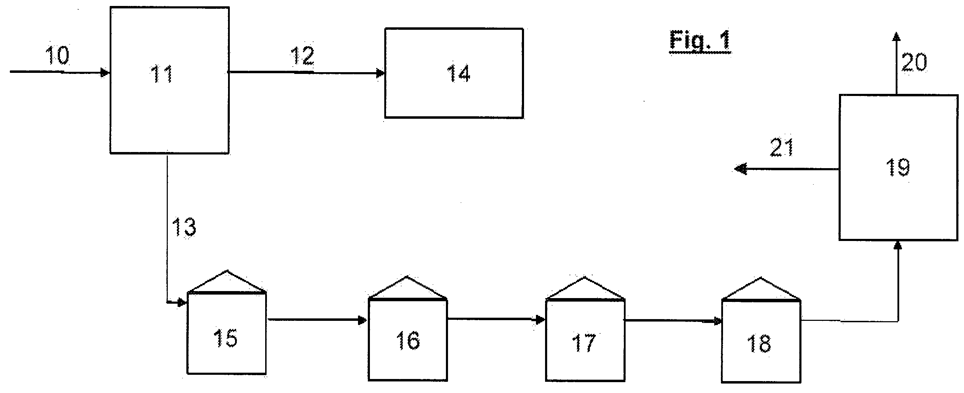

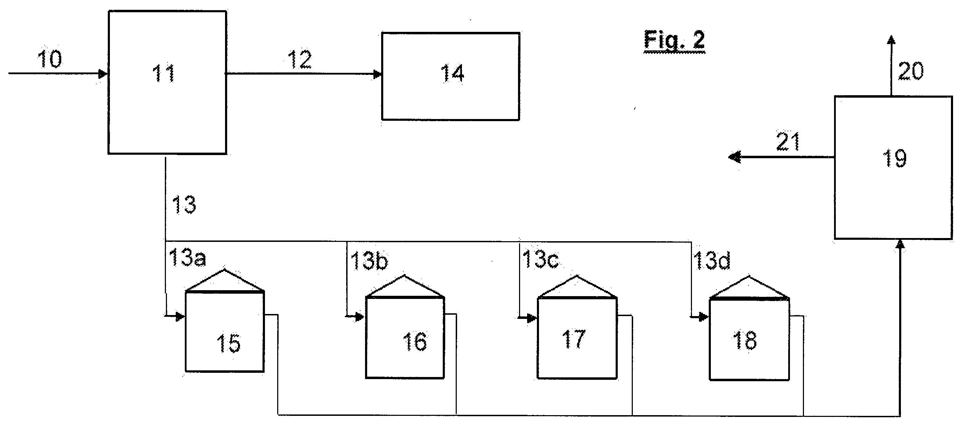

Wie in Figur 1 dargestellt wird das aus der Fermentation 10 stammende Biogas einer Biogasaufbereitungsanlage 11 zugeleitet, das den im wesentlichen aus Methangas bestehenden Produktgasstrom 12 von dem Abgasstrom 13, der im wesentlichen CO₂ enthält, trennt. Der Produktgasstrom kann in das Gasnetz 14 eingespeist werden. Der Abgasstrom wird entsprechend der Anordnung in Fig. 1 nacheinander in einen Substratlagerbehälter und in den Gärrestbehälter 16, 17 und 18 geleitet, die nacheinander durchströmt werden, bevor der Abgasstrom einer thermischen oder katalytischen Oxidationsstufe 19 zugeführt wird (serielle Anordnung). Die in Fig. 2 dargestellte Anordnung unterscheidet sich von der vorbeschriebenen Anordnung dadurch, dass der Abgasstrom 13 in mehrere Teilströme 13a, 13b, 13c und 13d aufgeteilt wird, die jeweils separat den Behältern 15 bis 18 zugeleitet werden (parallele Anordnung). Diese Behälter besitzen neben einer Gaszufuhrleitung eine Gasableitung, wobei die Gasableitungen aus den unterschiedlichen Behältern 15, 16, 17 und 18 in eine gemeinsame Leitung münden, die zur thermischen oder katalytischen Oxidationsstufe 19 führt. Die aus der thermischen oder katalytischen Oxidationsstufe erhaltenen Abgase 20 können in die Atmosphäre entlassen oder einer weiteren Behandlung oder Nutzung zugeführt werden. Die bei der thermischen oder katalytischen Oxidationsstufe entstehende Wärme 21 kann zur Deckung des Wärmebedarfs der Biogasanlage und ggf. externer Wärmeverbraucher genutzt werden.

Gleichgültig ob man eine Anlage gemäß Fig. 1 oder Fig. 2 wählt, lässt sich vorteilhafterweise die Menge brennbarer und hoch entzündlicher Gase, die in den Substratlagerbehältern und Gärrestbehältern entstehen, auf einen Gehalt minimieren, der nicht mehr explosiv ist. Die geschlossene Ausbildung sämtlicher Behälter 15 bis 18 vermeidet diffuse Gasemission. Die Emission von Methan wird durch die Nachoxidation vermieden. Die gesamte Anlage und Prozesssteuerung ist einfach aufgebaut und erfüllt die gesetzlichen Auflagen.

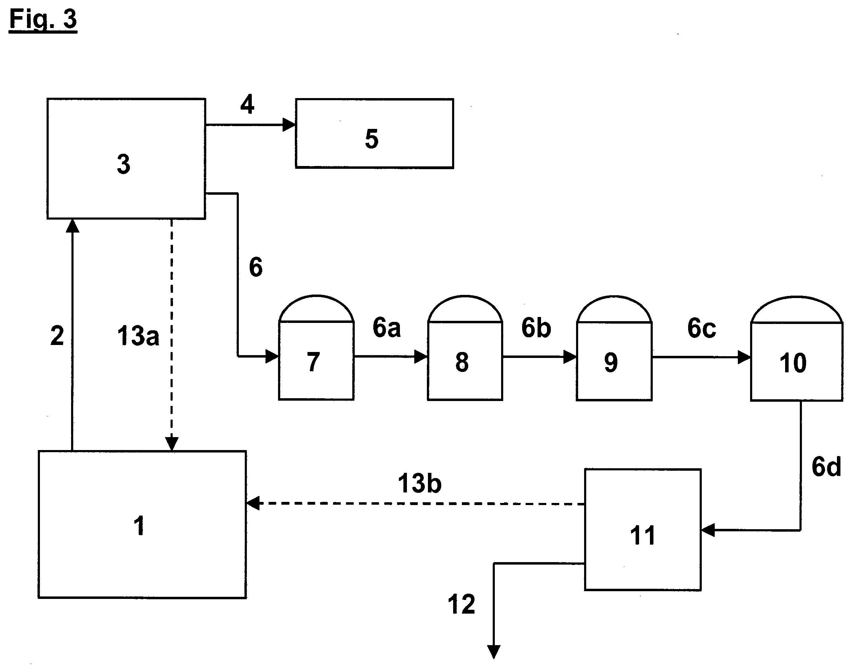

Das in Figur 3 dargestellte Ausführungsbeispiel zeigt, wie eine Biogasanlage mit 6,5 MW Leistung, die nach dem in Figur 1 dargestellten erfindungsgemäßen Verfahren mit serieller Durchleitung der Abgasströme arbeitet, in der realen technischen Durchführung ausgestaltet werden kann.

Das im Biogasfermentationsreaktor 1 produzierte Biogas gelangt über die Gasleitung 2 (Volumenstrom 1.200 m³/h, Methangehalt 53 %) zur Biogasaufbereitung 3. Nach der Biogasaufbereitung (Entschwefelung, Kohlendioxidabtrennung über DWA) gelangt ein Produktgasstrom (Volumenstrom 643 m³/h, Methangehalt 96 %) über Rohrleitung 4 zur Produktgaseinspeisungseinheit 5. Der hauptsächlich aus dem Inertgas CO₂ bestehende Abgasstrom wird über Rohrleitung 6 (Volumenstrom 572 m³/h, Methangehalt 3,3 %) zur Inertisierung nacheinander durch die gasdicht verschlossenen Gärrestbehälter 7, 8 und 9 sowie anschließend durch den gasdicht verschlossenen Substratlagerbehälter 10 geleitet. Zwischen den Gärrestbehältern 7,8 und 9 treten die Abgasströme 6a (Volumenstrom 580 m³/h) und 6b (Volumenstrom 588 m³/h), zwischen letztem Gärrestbehälter 9 und Substratlagerbehälter 10 der Abgasstrom 6c (Volumenstrom 596 m³/h, Methangehalt 5,3 %) und nach dem Substratlagerbehälter 10 der Abgasstrom 6d (Volumenstrom 601 m³/h, Methangehalt 5,8 %) auf. Abgasstrom 6d gelangt über eine Rohrleitung in die FLOX Brenner- und Kesselanlage 11, in der die flammenlose Oxidation erfolgt. Aus der FLOX Brenner- und Kesselanlage 11 wird das Restabgas über Leitung 12 in die Umgebung abgeführt. Sowohl in der Biogasaufbereitungsanlage 3 wie auch in der FLOX Brenner- und Kesselanlage 11 entsteht Wärme, die auf den Wegen 13a und 13b erneut der Biogasanlage zugeführt wird. Dabei wird über Wärmetauscher ein Wärmespeicher (Pufferspeicher) geladen, aus dem die Wärme für die Biogasanlage entnommen wird. Übertragungmedium ist dabei i.d.R. Wasser oder ein Wasser/Glykolgemisch.

Für die einzelnen Gasströme der in Figur 3 beschriebenen Biogasanlage wurden die in Tabelle 1 zusammengefassten Kenngrößen ermittelt.

**Tabelle 1: Kenngrößen für die Gasströme der beschriebenen 6,5 MW Biogasanlage**

| **Biogas:** | | |
|---|---|---|
| Volumenstrom | 1.200 | m³/h |
| Methangehalt | 53 | Vol-% |
| Methanvolumenstrom | 636 | m³/h |

| **Interne Entschwefelung:** | | |
|---|---|---|
| Luftvolumenstrom | 15 | m³/h |
| Sauerstoffvolumenstrom Biogas | 3,15 | m³/h |
| Stickstoffvolumenstrom Biogas | 11,85 | m³/h |
| Sauerstoffgehalt Biogas | 0,26 | Vol-% |
| Stickstoffgehalt Biogas | 0,98 | Vol-% |
| Biogas-/ Luftvolumenstrom gesamt | 1.215 | m³/h |
| | | |

| **Rohbiogas nach Entschwefelung** | | |
|---|---|---|
| Volumenstrom | 1.215 | m³/h |
| Methangehalt | 52,3 | Vol-% |
| | | |

| **Biogasaufbereitung (DWA):** | | |
|---|---|---|
| Methanschlupf | 3,0 | Vol-% |
| **Volumenstrom Produktgas** | 643 | m³/h |
| Methangehalt Produktgas | 96,0 | Vol-% |
| Abscheideanteil Sauerstoff | 50,0 | % |
| Abscheideanteil Stickstoff | 50,0 | % |
| Sauerstoffvolumenstrom Produktgas | 1,58 | m³/h |
| Sauerstoffvolumenstrom Produktgas | 5,93 | m³/h |
| Sauerstoffgehalt Produktgas | 0,25 | Vol-% |
| Stickstoffgehalt Produktgas | 0,92 | Vol-% |
| **Volumenstrom Abgas** | 572 | m³/h |
| Volumenstrom Methan im Abgas | 19,1 | m³/h |
| Sauerstoffvolumenstrom Abgas | 1,58 | m³/h |
| Stickstoffvolumenstrom Abgas | 5,93 | m³/h |
| Sauerstoffgehalt Abgas | 0,28 | Vol-% |
| Stickstoffgehalt Abgas | 1,04 | Vol-% |
| Methangehalt Abgas | 3,33 | Vol-% |
| | | |

| **Gärrestbehälter als Puffer für Abgas aus DWA:** | | |
|---|---|---|
| | | % von o.g. |
| Biogasbildung | 2,0 | Biogasproduktion |
| Biogasbildung | 24,0 | m³/h |
| H₂S-Gehalt im gebildeten Biogas, max | 300,00 | ppm |
| Methanbildung | 12,7 | m³/h |
| Methaneintrag über Abgas DWA | 19,1 | m³/h |
| Methananteil | 5,33 | Vol-% |
| erforderlicher Arbeits-Gasspeichervolumen | 1.622 | m³ |
| | | |

| **Substratlagerbehälter als Puffer für Abgas aus DWA:** | | |
|---|---|---|
| Biogasbildung | 5,0 | m³/h |
| H₂S-Gehalt im gebildeten Biogas, max | 300,00 | ppm |
| Methanbildung | 2,7 | m³/h |
| Methaneintrag aus Gärrestbehälter | 43,1 | m³/h |
| Methananteil gesamt | 5,84 | Vol-% |
| | | |

| **Abgas aus Substratlagerbehälter zur Abgasbehandlung:** | | |
|---|---|---|
| Volumenstrom | 601 | m³/h |
| H₂S-Gehalt | 14,47 | ppm |
| Methananteil gesamt | 5,84 | Vol-% |
| Sauerstoffgehalt Abgas | 0,26 | Vol-% |
| Stickstoffgehalt Abgas | 0,99 | Vol-% |
| Temperatur min. 5 | 5 | °C |
| Temperatur max. | 60 | °C |
| Feuchtegehalt max. | 100 | % |

Die Volumenströme für das zur Inertisierung benutzte Abgas wurden nach den Angaben in Tabelle 2 berechnet. Die Nummerierung der Komponenten der Biogasanlage erfolgte analog Figur 3.

**Tabelle 2: Abgasströme, die zur Inertisierung von Gärrestbehältern und Substratlagerbehälter verwendet wurden**

| **Rohrleitung von DWA 3 zum Gärrestbehälter 7** | | |
|---|---|---|
| Volumenstrom | 572 | m³/h |
| Dimension | DN 150 | |
| Innendurchmesser | 152,2 | mm |
| Strömungsgeschwindigkeit | 8,74 | m/s |
| Druckverlust | 7,3 | mbar |

| **Rohrleitung vom Gärrestbehälter 7 zum Gärrestbehälter 8** | | |
|---|---|---|
| Volumenstrom | 580 | m³/h |
| Dimension | DN 300 | |
| Innendurchmesser | 302,6 | mm |
| Strömungsgeschwindigkeit | 4,41 | m/s |
| Druckverlust | 0,3 | mbar |
| | | |

| **Rohrleitung vom Gärrestbehälter 8 zum Gärrestbehälter 9** | | |
|---|---|---|
| Volumenstrom | 588 | m³/h |
| Dimension | DN 300 | |
| Innendurchmesser | 302,6 | mm |
| Strömungsgeschwindigkeit | 6,61 | m/s |
| Druckverlust | 0,7 | mbar |
| | | |

| **Rohrleitung vom Gärrestbehälter 9 zum Substratlagerbehälter 10** | | |
|---|---|---|
| Volumenstrom | 596 | m³/h |
| Dimension | DN 300 | |
| Innendurchmesser | 302,6 | mm |
| Strömungsgeschwindigkeit | 2,51 | m/s |
| Druckverlust | 0,15 | mbar |
| | | |

| **Rohrleitung vom Substratlagerbehälter 10 zum FLOX-Brenner 11** | | |
|---|---|---|
| Volumenstrom | 601 | m³/h |
| Dimension | DN 200 | |
| Innendurchmesser | 206,5 | mm |
| Strömungsgeschwindigkeit | 5,39 | m/s |
| Druckverlust | 1,25 | mbar |

In der Massenbilanz von gebildeten Gasen aus der gesamten Anlage ergeben sich die in Tabelle 3 zusammengefassten Gasmengen mit ihren entsprechenden anteilsmäßigen Zusammensetzungen an den Gasen Methan, Sauerstoff, Stickstoff, Schwefelwasserstoff und Kohlendioxid. Es wurde hierbei ein Anlagenbetrieb von 365 Tagen pro Jahr mit jeweils 24 h pro Tag angenommen.

**Tabelle 3: Gasmassenbilanz und Gaszusammensetzung für die 6,5 MW Biogasanlage aus Figur 3**

| **Gas** | **Menge [m³/a]** | **CH₄ [%]** | **O₂ [%]** | **N₂ [%]** | **H₂S [ppm]** | **CO₂ [%]** |
|---|---|---|---|---|---|---|
| Rohbiogas | 10.643.400 | 52,3 | 0,26 | 0,98 | <300 | 46,42 |
| Produktgas | 5.629.395 | 96,0 | 0,25 | 0,92 | 0 | 2,83 |
| Inertgas | 5.014.005 | 3,3 | 0,28 | 1,04 | 0 | 95,36 |
| Gasbildung Gärrestbehälter | 210.240 | 53 | 0 | 0 | <300 | 47,00 |
| Gasbildung Substratlagerbehälter | 43.800 | 53 | 0 | 0 | <300 | 47,00 |
| Gasverwertung FLOX | 5.268.045 | 5,84 | 0,26 | 0,99 | 14,47 | 92,91 |

Es zeigte sich, dass das erfindungsgemäße Verfahren, bei dem der Abgasstrom, der aus der Biogasaufbereitung stammt, vor der abschließenden Oxidation durch die geschlossen ausgebildeten Gärrestbehälter und Substratlagerbehälter geleitet wird, in einer erfindungsgemäß ausgebildeten Biogasanlage geeignet ist, um sowohl den Explosionsschutz zu gewährleisten als auch in den Gärrestbehältern und Substratlagerbehältern zusätzlich gebildetes Biogas energetisch zu nutzen. Das Abgas aus der Biogasaufbereitung wies eine Konzentration von 3,3 Vol% Methan und 95,4 Vol% Kohlendioxid auf, so dass es aufgrund seiner Zusammensetzung sehr gut als Gas zur Inertisierung von Gärrestbehältern und Substratlagerbehälter geeignet war. Die auftretenden Abgasströme waren von ihrem Volumen her völlig ausreichend, um das in den Gärrestbehältern und dem Substratlagerbehälter entstehende Biogas zu inertisieren, so dass der Methangasanteil nach serieller Durchströmung der 3 Gärrestbehälter bei 5,33 Vol% lag und nach zusätzlicher Durchströmung des Substratlagerbehälters bei 5,84 Vol%. In jedem Fall lag also die Methankonzentration in den freien Gasvolumina innerhalb der Vorlage- und Gärrestbehälter weit unterhalb der Grenze von 17,5 %, ab der in Kontakt mit Luft ein explosives Gemisch entstehen könnte. Innerhalb der abgeschlossenen Vorlage- und Gärrestbehälter wird eine Explosionsgefahr ohnehin durch den vorhandenen hohen CO₂-Anteil, das als Inertgas wirkt, gebannt. Das in den Gärrestbehältern und im Vorlagenbehälter gebildete Biogas machte mit einem Volumen von 254.040 m³/a einen Anteil von 2,4 % des im Fermentationsreaktor gebildeten Biogases aus und konnte durch die anschließende Oxidation im FLOX-Brenner zusätzlich als thermische Energie zum Erwärmen des Biogasfermenters genutzt werden. Durch die geschlossen ausgebildeten Gärrestbehälter und den Substratlagerbehälter sowie die flammenlose Oxidation des Restabgases wurde eine Emission von Methan und Kohlendioxid in die Umwelt vermieden, so dass auch Klimaschutzaspekte bei dem erfindungsgemäßen Verfahren und in der erfindungsgemäßen Biogasanlage berücksichtig wurden.

## Patentansprüche

1. Verfahren zur Biogasaufbereitung, bei dem das aus einer Fermentation stammende Biogas in einen direkt nutzbaren, im wesentlichen aus Methangas bestehenden Gasstrom und einen Störstoffe enthaltenden Abgasstrom getrennt wird, der thermisch oder katalytisch oxidiert wird, **dadurch gekennzeichnet, dass** der Abgasstrom vor der Oxidation durch geschlossen ausgebildete Substratlagerbehälter und/oder Gärrestbehälter durchgeleitet wird.

2. Verfahren nach Anspruch 1, **dadurch gekennzeichnet, dass** das Abgas die Substratlagerbehälter und/oder Gärrestbehälter nacheinander durchströmt oder in Teilmengen separiert wird, welche jeweils den Substratlagerbehälter und/oder Gärrestbehälter getrennt zugeleitet werden.

3. Verfahren nach Anspruch 1 oder 2, **dadurch gekennzeichnet, dass** das Abgas abschließend verbrannt und die Verbrennungswärme zur Deckung des Wärmebedarfs der Biogasanlage und ggf. externer Wärmeverbraucher benutzt wird.

4. Biogasanlage mit einem oder mehreren Fermentationsbehältern und einer Biogasaufbereitungsanlage zur Trennung von Biogas und Abgas, wobei eine Abgasführung eine abschließende thermische oder katalytische Oxidationsstufe aufweist, sowie mindestens mit einem Substratlagerbehälter und/oder Gärrestbehälter, **dadurch gekennzeichnet, dass** die Abgasführung durch die Substratlagerbehälter und/oder Gärrestbehälter geleitet wird, die bis auf eine Gaszufuhr und eine Gasabfuhr geschlossen ausgebildet sind.

5. Biogasanlage nach Anspruch 4, **dadurch gekennzeichnet, dass** die Substratlagerbehälter und/oder Gärrestbehälter hinsichtlich der Abgasführung in Serie oder parallel geschaltet sind.

6. Biogasanlage nach Anspruch 4 oder 5, **dadurch gekennzeichnet, dass** die Biogasaufbereitung über eine Gaswäsche mittels Waschflüssigkeit (z.B. Wasser, Polyglykol, MEA-Wäsche), über eine Druckwechseladsorption (DWA), über ein Membrabtrennverfahren (nass oder trocken), oder eine Kohlendioxidverflüssigung erfolgt.

## Claims

1. A method for treating biogas, in which the biogas originating from a fermentation is separated into a directly utilizable stream of gas essentially consisting of methane gas and into a waste gas stream containing contaminants which is thermally or catalytically oxidized, **characterized in that** prior to the oxidation, the waste gas stream is conducted through closed substrate storage tanks and/or fermentation residue tanks.

2. The method according to claim 1, **characterized in that** the waste gas flows through the substrate storage tanks and/or through the fermentation residue tanks one after the other, or it is separated into parts, each of which is fed separately into the substrate storage tanks and/or into the fermentation residue tanks.

3. The method according to claim 1 or 2, **characterized in that** the waste gas is subsequently burned, and the heat of combustion is utilized in order to meet the heat requirement of the biogas plant and, if applicable, of external heat consumers.

4. A biogas plant having one or more fermentation tanks and having a biogas treatment plant for separating biogas from waste gas, wherein a waste-gas ducting system has a subsequent thermal or catalytic oxidation state, as well as having at least one substrate storage tank and/or fermentation residue tank, **characterized in that** the waste gas ducting system is passed through the substrate storage tanks and/or through the fermentation residue tanks which are closed except for a gas inlet and a gas outlet.

5. The biogas plant according to claim 4, **characterized in that** the substrate storage tanks and/or the fermentation residue tanks are connected in series or in parallel with respect to the waste gas ducting system.

6. The biogas plant according to claim 4 or 5, **characterized in that** the biogas treatment is carried out by means of gas scrubbing employing a scrubbing liquid (for example water, polyglycol, MEA scrubbing), by means of pressure swing adsorption (DWA), by means of a membrane separation process (wet or dry) or by means of carbon dioxide liquefaction.

## Revendications

1. Procédé destiné à traiter du biogaz, lors duquel on sépare le biogaz émanant d'une fermentation en un flux gazeux directement utilisable, constitué principalement de méthane et un flux de gaz perdu contenant des matières perturbatrices que l'on fait oxyder par voie thermique ou catalytique, **caractérisé en ce qu'**avant l'oxydation, on fait passer le flux de gaz perdus à travers des réservoirs de stockage de substrats et/ou des réservoirs de digestats.

2. Procédé selon la revendication 1, **caractérisé en ce que** le gaz perdu passe successivement à travers les réservoirs de stockage de substrats et/ou les réservoirs de digestats ou **en ce qu'**on le sépare en quantités partielles, qui sont respectivement amenées séparément dans les réservoirs de stockage de substrats et/ou les réservoirs de digestats.

3. Procédé selon la revendication 1 ou 2, **caractérisé en ce que** pour finir, on fait brûler le gaz perdu et **en ce qu'**on utilise la chaleur de combustion pour couvrir les besoins en chaleur de l'installation de biogaz ou le cas échéant, de consommateurs de chaleur externes.

4. Installation de biogaz avec un ou plusieurs réservoirs de fermentation et une installation de traitement de biogaz, destinée à séparer le biogaz et le gaz perdu, une conduite de gaz perdu comportant une étape finale d'oxydation thermique ou catalytique, ainsi qu'avec au moins un réservoir de stockage de substrats et/ou un réservoir de digestats, **caractérisée en ce que** la conduite de gaz perdu est dirigée à travers les réservoirs de stockage de substrats et/ou les réservoirs de digestats, qui mis à part un alimentation en gaz et une évacuation de gaz sont conçus en version fermée.

5. Installation de biogaz selon la revendication 4, **caractérisée en ce que** les réservoirs de stockage de substrats et/ou les réservoirs de digestats sont montés en série ou en parallèle par rapport à la conduite de gaz.

6. Installation de biogaz selon la revendication 4 ou 5, **caractérisée en ce que** le traitement du biogaz s'effectue par l'intermédiaire d'une épuration du gaz au moyen d'un liquide de lavage (par ex. de l'eau, un polyglycol, un lavage MEA), par l'intermédiaire d'une adsorption modulée en pression (DWA), par l'intermédiaire d'un procédé de séparation sur membrane (humide ou à sec) ou d'une fluidification au dioxyde de carbone.
